# EUROPEAN PATENT APPLICATION

(11) **EP 1 745 804 A1**
(43) Date of publication of application: **24.01.2007**
(21) Application number: 06116925.6
(22) Date of filing: 10.07.2006
(51) Int. Cl.: A61L 2/04, A23L 3/10, B65B 55/02

(54) **Apparatus for sterilising packaged food products**

(30) Priority: 19.07.2005 IT MI20051368
(71) Applicant: Galvanin, Bruno, 30123 Venezia (IT)
(72) Inventor: Galvanin, Bruno, 30123 Venezia (IT)
(74) Representative: Mittler, Enrico

(57) **Abstract**

It is described an apparatus for sterilising packaged food products comprised of an autoclave (1) which can be filled at least partially with heatable liquid (8) for fully immersing packaged food products (7) housed therein, said autoclave (1) comprising means (9) for the controlled heating and cooling of said liquid and means (11) for circulating said liquid (8) within the autoclave (1).

## Description

The present invention relates to an apparatus for sterilising packaged food products.

Normally, packaged food products are sterilised in large hermetic containers comprising nozzles, from which hot pressurised liquid (generally water) is issued.

The nozzles are pointed towards the packages to be sterilised and the liquid which is issued is generally mixed with steam to create a proper cloud capable of reaching all the packages.

The main problem of this type of sterilisation system is ensuring that the whole container is at the same temperature, i.e. equal to or higher than the sterilisation temperature, so as to guarantee the genuineness of the food product to be preserved in time and that all product packages to be sterilised are subject to jets of liquid in the same measure.

The aforesaid problem can only be limited by increasing the number of nozzles.

Actually, it is not rare to find rotted packaged food products despite the remote best-before date. Usually, these are the products whose packages are positioned in the central zone of the sterilisation containers which is less steeped by the sprays from the nozzles.

It is the object of the present invention to make an apparatus which ensures the sterilisation of packaged food products with higher safety.

According to the invention, such object is achieved by an apparatus for sterilising packaged food products, characterised in that it consists of an autoclave which can be filled at least partially with heatable liquid for fully immersing packaged food products housed within, said autoclave comprising means for the controlled heating and cooling of said liquid and means for circulating said liquid within said autoclave.

The complete immersion of the packaged food products and the induced circulation of the liquid used as heat vector allow a uniform distribution of the temperature in the autoclave and therefore an equal sterilisation of all products.

These and other features of the present invention will be further explained in the following detailed description of an exemplary embodiment thereof shown by way of non-limitative example in the attached drawings, in which:
figure 1 shows a schematic longitudinal sectional view of an apparatus according to the present invention;
figure 2 shows a sectional view taken along line II-II in figure 1;
figure 3 shows a sectional view taken along line III-III in figure 1;
figure 4 shows a longitudinal sectional view similar to that of figure 1 but with reversed liquid circulation;
figure 5 shows a sectional view taken along line V-V in figure 4;
figure 6 shows a sectional view taken along line VI-VI in figure 4.

An apparatus for the sterilisation of packaged food products according to the present invention is comprised of an autoclave 1 hermetically closed by a removable lid 2 by means of a mechanism 13 and comprising an internal chamber 3 and an external chamber 4 separated by walls 5 which have perforated portions 6.

The autoclave 1 shown in the figures has a horizontal axis, but it is also possible to use a vertical axis autoclave 1 for the sterilisation process.

Packaged food products 7 immersed in a heat vector liquid 8 (generally water or aqueous solution) heated by serpentines 9 with attachments 10 contained in the external chamber 4 are housed in the internal chamber 3 of the autoclave 1.

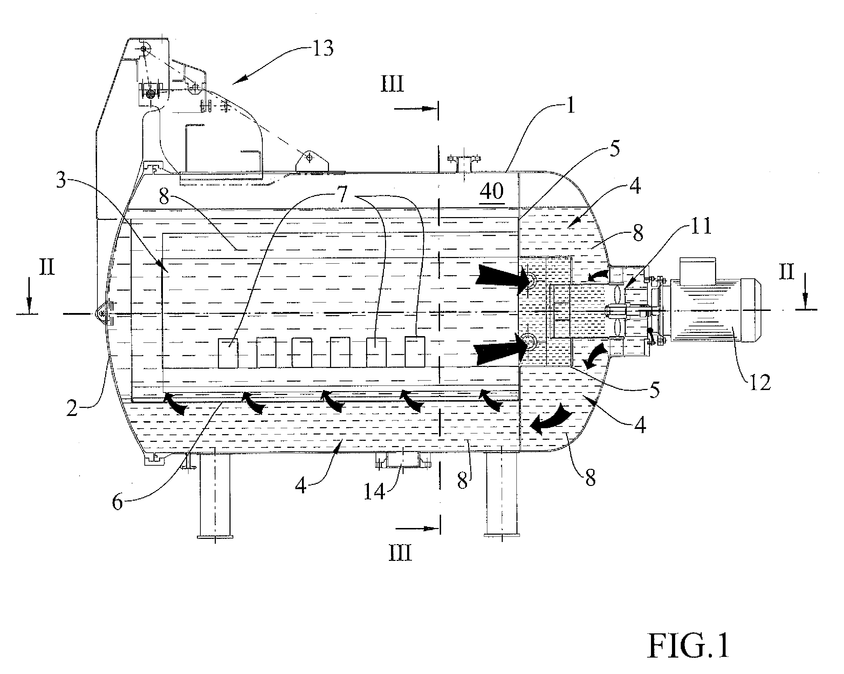

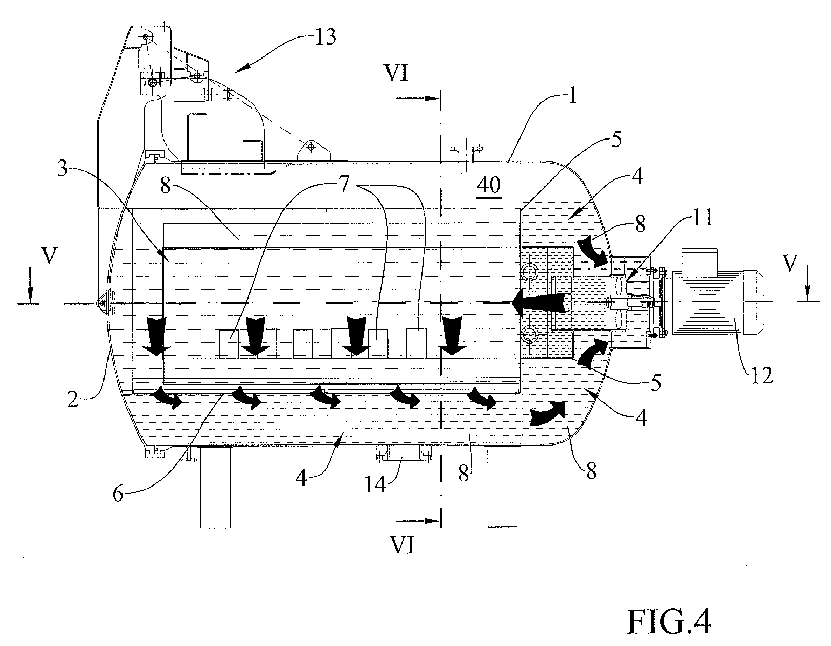

A reversible helical pump 11 controlled by an external motor 12 allows said liquid 8 to circulate from said internal chamber 3 to the external chamber 4 (figure 1) and vice versa (figure 4) through said perforated portions 6 of the walls 5.

The liquid 8 may be immersed in the autoclave 1 and discharged therefrom through an inlet/outlet attachment 14.

The autoclave 1 is filled sufficiently but not completely to ensure the complete immersion of the packaged food products 7 to be sterilised, a variable air cushion 40 therefore remaining in its upper part with a level sensor (not shown) to indicate the level of liquid 8 contained in the autoclave 1.

The apparatus also comprises temperature sensors 16 for the continuous monitoring of the temperature within the autoclave 1.

As concerns operation, we will consider the autoclave 1 empty with the lid 2 removed.

Once the packaged food products 7 are positioned in the internal chamber of the autoclave 1, the lid 2 is hermetically closed by operating the mechanism 13 and the autoclave is filled with heat vector liquid 8.

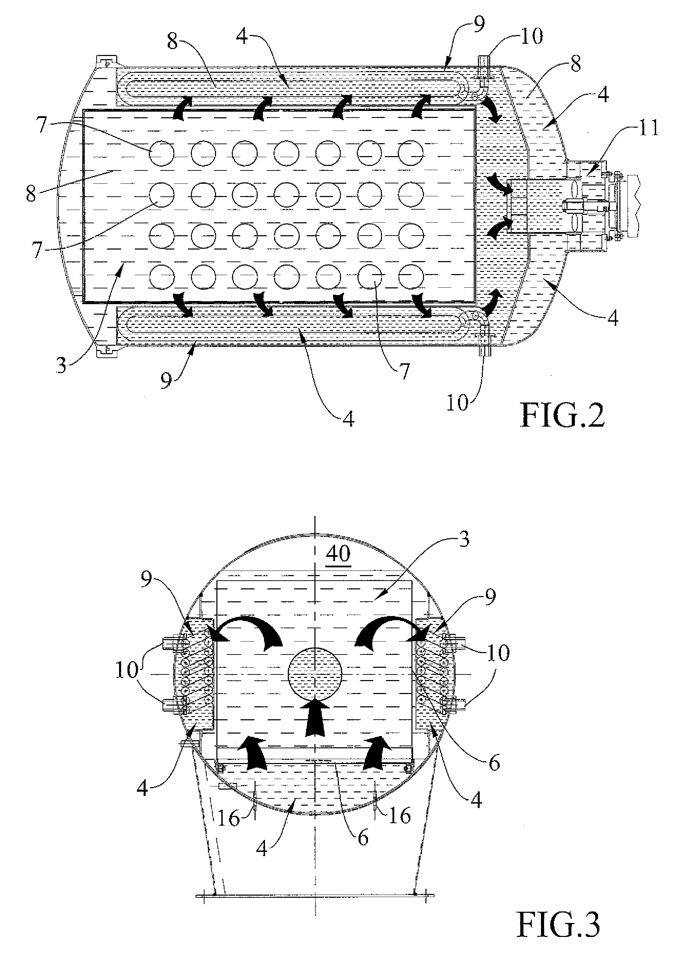

After checking that all the packages 7 are immersed, the liquid 8 is heated by running hot water or steam in the serpentines 9 and the pump 11 is operated for inducing a circulation of liquid, for example, as shown by the arrows in figures 1-3. The liquid temperature increases with the progression of the circulation of the liquid itself. The heating gradient is programmable according to the material to be treated.

The liquid must reach a predetermined sterilisation temperature in all internal points of the autoclave 1 to sterilise the food products in the packages 7. Some sensors 16 allow to understand if this occurs.

A control unit (not shown) continuously indicates the internal temperature to the user through specific sensors 16 arranged inside the autoclave and consequently reacts to the determinations and if, after the initial heating of the fluid 8, there are drops of temperature, said unit controls the flow of further heating liquid through the serpentines 9.

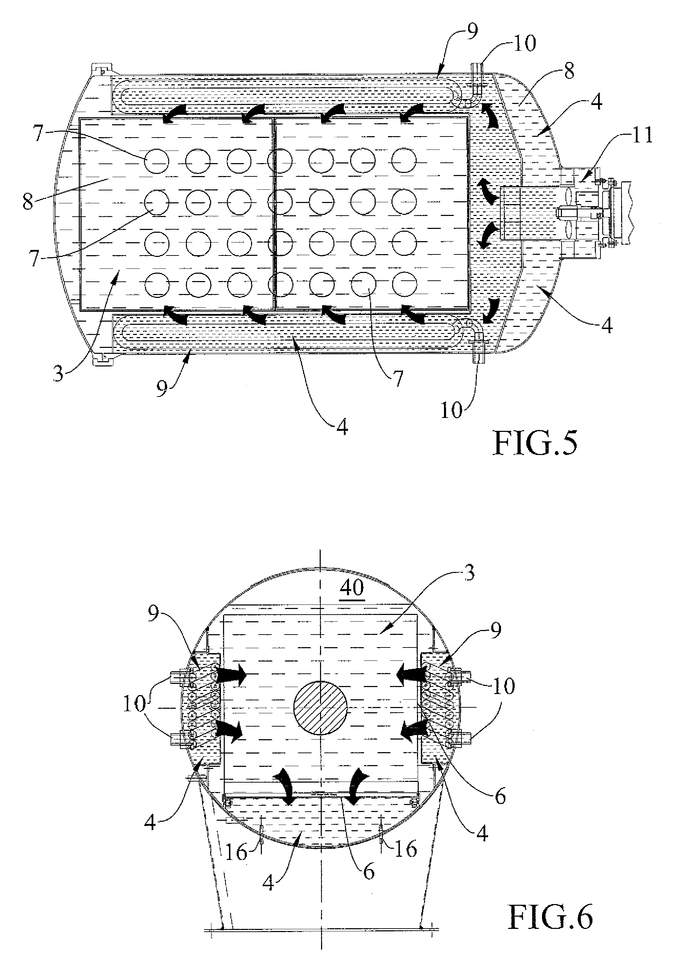

To allow a uniform distribution of the temperature in the autoclave 1, and therefore the sterilisation of the packages, the control unit periodically controls the flow of rotation of the pump 11 and therefore the circulation of the liquid 9 to reverse (figure 4-6).

After a certain time, previously defined to ensure the safe sterilisation of the packages 7, a cooling cycle of the liquid starts by flowing cold air within the serpentine 9 and the pump 11 is operated to induce a circulation of liquid as shown for example by the arrows in fig. 1-3. The cooling gradient is programmable according to the material to be treated. At this point, the pump 11 is stopped, the liquid 8 is drained through the attachment 14 and the sterilised packages 7 are extracted from the autoclave 1 by removing the lid 2.

The apparatus according to the present invention is very flexible and may be adapted to variable loads: the air cushion 40 may increase or decrease in volume according to the number of packages 7 to be sterilised existing in the autoclave 1. This allows to load the autoclave 1 also partially not having to consume the liquid 8 in excess. It will suffice to introduce an amount of liquid 8 sufficient to fully immerge the packages 7 to be sterilised.

## Claims

1. An apparatus for sterilising packaged food products, **characterised in that** it is comprised of an autoclave (1) which can be filled at least partially with heatable liquid (8) for fully immersing packaged food products (7) housed therein, said autoclave (1) comprising means (9) for the controlled heating and cooling of said liquid and means (11) for circulating said liquid (8) within the autoclave (1).

2. An apparatus according to claim 1, **characterised in that** said autoclave (1) is comprised of an internal chamber (3) and an external chamber (4) reciprocally separated by walls (5) having perforated portions (6) for the circulation and distribution of the liquid (8).

3. An apparatus according to claim 1, **characterised in that** said means (9) for heating and cooling the liquid are comprised of at least one serpentine (9) in which further flows either hot temperature fluid to heat the liquid (8) contained in the autoclave (1) or conversely cold water to cool the liquid (8) contained in the autoclave (1).

4. An apparatus according to claim 3, **characterised in that** said at least one serpentine (9) is contained in said external chamber (4).

5. An apparatus according to claim 1, **characterised in that** said liquid circulation means are comprised of a reversible pump (11) contained in the autoclave (1) and fed by an external motor (12).

6. An apparatus according to claim 1, **characterised in that** said pump (11) is operable in reversed direction to reverse said circulation of fluid (8).

7. An apparatus according to claim 1, **characterised in that** said autoclave (1) is provided with a removable lid (2).

8. An apparatus according to claim 1, **characterised in that** it comprises sensors (16) for detecting the temperature in a plurality of zones of the autoclave (1) so as to guarantee a complete and continuous monitoring of the temperature distribution in the autoclave (1) itself.

9. An apparatus according to claim 1, **characterised in that** said heatable liquid (8) is water.

10. An apparatus according to claim 1, **characterised in that** said heatable liquid (8) is an aqueous solution.

11. An apparatus according to claim 1, **characterised in that** said liquid (8) within the autoclave 1 is topped by a variable air cushion (40) according to the level of the liquid (8), i.e. the quantity of packaged food products (7) to be sterilised which must be completely immersed in said liquid (8).
